# EUROPEAN PATENT APPLICATION

(11) **EP 4 080 208 A1**
(43) Date of publication of application: **26.10.2022**
(21) Application number: 21170297.2
(22) Date of filing: 23.04.2021
(51) Int. Cl.: G01N 33/50, G01N 33/543, G01N 33/58

(54) **QUANTIFICATION OF SUCCESSFUL ENCAPSULATION INTO MICROFLUIDIC COMPARTMENTS**

(71) Applicant: ECOLE POLYTECHNIQUE FEDERALE DE LAUSANNE (EPFL), 1015 Lausanne (CH); European Molecular Biology Laboratory, 69117 Heidelberg (DE)
(72) Inventor: MERTEN, Christoph, 1028 Preverenges (CH); GRAB, Anna, 74933 Neidenstein (DE)
(74) Representative: Kuttenkeuler, David

(57) **Abstract**

The invention provides a method for quantifying the number of target entities that were successfully encapsulated into a microfluidic compartment such as a microfluidic droplet. The invention is predicated upon a continuous quantifiable detectable signal corresponding to the quantity of entities encapsulated in a microfluidic compartment. The present invention is useful in the context of high throughput microfluidic screening approaches which require thorough signal normalization in order to reliably improve signal to noise ratio and to reliably identify screening hits.

## Description

### FIELD OF THE INVENTION

The invention provides a method for quantifying the number of target entities that were successfully encapsulated into a microfluidic compartment such as a microfluidic droplet. The invention is predicated upon a continuous quantifiable detectable signal corresponding to the quantity of entities encapsulated in a microfluidic compartment. The present invention is useful in the context of high throughput microfluidic screening approaches which require thorough signal normalization in order to reliably improve signal to noise ratio and to reliably identify screening hits.

### DESCRIPTION

Two phase microfluidics holds great potential for high throughput screening applications. In these systems, aqueous samples surrounded by oil serve as miniaturized test tubes (Shembekar N et al., Lab on a Chip 16(8):1314-1331.; Shembekar N et al., Cell Rep 22(8):2206-2215; Mathur L et al., Small:e1904321.). The aqueous microcompartments can be either small surfactant stabilized droplets (emulsions, typically with picoliter volumes), larger plugs that completely fill a channel or tubing and that are spaced out by oil (without the need for stabilizing surfactants, typically with nanolitre volumes) or even flow segments within a channel or tubing exceeding microliter volumes. The encapsulation of single cells into droplets allows screening of cell products such as antibodies at very high throughput, e.g. up to several hundred thousand samples per day. For example, individual antibody-secreting cells (e.g. hybridoma- or B-cells) can be encapsulated into droplets together with a recombinant drug target of interest, such as an enzyme. Addition of a fluorogenic substrate of the enzyme will then reveal which droplets contain a cell releasing antibodies which inhibit enzymatic activity and facilitate the specific sorting of these. However, for many screening applications, it would be very useful to co- encapsulate (in addition to the antibody secreting cell) another reporter cell, rather than a recombinant enzyme. This reporter cell could mediate a fluorescence signal (e.g. expression of a reporter enzyme which can easily be detected in droplets; such as β-Gal) upon the desired effect of an antibody, such as the stimulation or inhibition of cellular pathways upon antibody binding of G protein-coupled receptors (GPCRs). GPCRs are the targets of most best-selling drugs and about 40% of all prescription pharmaceuticals.

A crucial step for this kind of screens is the generation of droplets hosting both cell types, optionally at the single cell level. Non-deterministic cell encapsulation is limited by Poisson statistics, meaning that the number of cells per droplets varies significantly. Alternative deterministic encapsulation procedures have been described, however, until today the reliable co-encapsulation of two different cell types has not been demonstrated, especially not at single-cell level or at high throughput. Therefore, cell-based assays requiring the co-encapsulation of two different cell types at the single cell level (as required for drug screening; e.g. one B-cell secreting antibodies and another reporter cell indicating the effect of the antibody on a cellular target) can hardly be performed in droplets. This is due to the fact that the cell occupancy in each droplet cannot be controlled tightly, including controlled co-encapsulation of cells and beads as required for antibody binding assays. Furthermore, the quantitative analysis of antibody binding is challenging, as the bead (and any fluorescently labelled objects bound to it) can float out of the focal plane and/or out of the centre of the laser spot (having the highest intensity).

When performing a biological assay within an aqueous microcompartment, it is important to normalize the assay signal (e.g. apoptosis activity, viability, etc.) to the number of encapsulated cells per sample. Otherwise, samples with higher cell numbers (note that the number of encapsulated cells cannot be controlled tightly and typically follows a Poisson distribution (Clausell-Tormos J, et al. 2008 Chem. Biol. 15(5):427-437.)) inevitably show a higher assay signal that strongly biases the readout. It is therefore of utmost importance to reliably measure the number of cells in each compartment. For small droplets with single or only a few cells this can be done by staining the cells with unspecific fluorescence dyes (Hu H, et al. 2015 Lab on a Chip 15(20):3989-3993). However, this approach fails for compartments containing many cells (e.g. cell clumps or at least cells in different optical layers eclipsing each other) or for large compartments in which even individual cells can be far outside the focal plane (resulting in a signal that is potentially below the detection limit).

Therefore, it is an object of the present invention to overcome the above limitations and to provide a procedure that allows to measure cell numbers accurately, even for large compartments with many encapsulated entities such as biological cells.

### BRIEF DESCRIPTION OF THE INVENTION

The approach of the present invention is based on staining the target entities to be encapsulated prior to encapsulation with enzyme-linked antigen binding molecules such as antibodies. Subsequent to washing steps the cells are encapsulated into aqueous compartments, together with all assay reagents and a fluorogenic substrate of the enzyme. After an incubation time sufficient to convert the fluorogenic substrate partially (non-endpoint measurement), the fluorescence of the entire aqueous compartment is measured and the obtained signal linearly correlates with the number of encapsulated cells. An innovative feature of this approach is that the cell number is determined indirectly: rather than imaging cells or measuring intracellular fluorescence (whose intensity depends on the focal plane), the invention suggest to measure the presence of fluorophores that are homogeneously distributed in the aqueous microcompartments, meaning that the signal can be determined totally independent of the cell position within the compartment. Furthermore, this type of assay is also applicable to cell clumps or cells in different optical layers and potentially even to microscopic tissue slices encapsulated into microcompartments.

Generally, and by way of brief description, the main aspects of the present invention can be described as follows:

In **a first aspect,** the invention pertains to a method for the quantification of the number of target entities encapsulated in a microcompartment, the method comprising the steps of:
- Providing a detection molecule comprising (i) a target binding site specific for the target entity and (ii) an enzymatic component capable of catalysing a chemical signalling reaction;
- Bringing into contact the detection molecule and the target entity to allow for a specific binding of the detection molecule to the target entity;
- Optionally, removing at least any unbound detection molecules;
- Encapsulating one or more (an unknown number of) target entities bound with detection molecules into one or more micro-compartments together with substrate molecules, wherein the substrate molecule is a substrate of the enzymatic component, and which when brought into contact with the enzymatic component renders the enzymatic component to catalyse the chemical signalling reaction; and
- Quantifying the detectable signal for each micro compartment and thereby quantifying the number of target entities encapsulated in each microcompartment.

In **a second aspect,** the invention pertains to a plurality of microcompartments, of which at least 5% of microcompartments have an aqueous phase comprising a target entity bound with detection molecules, wherein the detection molecule comprises (i) a target binding site specific for the target entity and (ii) an enzymatic component that is capable of catalysing a chemical signalling reaction; and an unbound substrate molecule dissolved within the aqueous phase and which is a substrate of the enzymatic component of the detection molecule, and which when brought into contact with the enzymatic component renders the enzymatic component to catalyse the chemical signalling reaction that generates a detectable signal.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a method for the quantification of the number of target entities encapsulated in a microcompartment, the method comprising the steps of:
- Providing a detection molecule comprising (i) a target binding site specific for the target entity and (ii) an enzymatic component capable of catalysing a chemical signalling reaction;
- Bringing into contact the detection molecule and the target entity to allow for a specific binding of the detection molecule to the target entity;
- Optionally, removing at least any unbound detection molecules;
- Encapsulating one or more (an unknown number of) target entities bound with detection molecules into one or more micro-compartments together with substrate molecules, wherein the substrate molecule is a substrate of the enzymatic component, and which when brought into contact with the enzymatic component renders the enzymatic component to catalyse the chemical signalling reaction; and
- Quantifying the detectable signal for each micro compartment and thereby quantifying the number of target entities encapsulated in each microcompartment.

In an alternative embodiment of the first aspect, the detection molecule is two detection molecules, namely a first detection molecule which comprises (i) a target binding site specific for the target entity and (ii) a binding site capable of being bound by a second detection molecule, wherein the second detection molecule comprises (x) a target binding site specific for the detection molecule, and (y) an enzymatic component capable of catalysing a chemical signalling reaction. In this second embodiment, a complex is formed between the target, the detection molecule and the second detection molecule which then may elicit a detectable signal. An illustrative example of this embodiment is a use of a primary antibody specifically binding a target protein, and a secondary antibody coupled to a signaling moiety, and which is specific for the primary antibody.

Hence, in an **alternative of the first aspect,** the invention pertains to a method for the quantification of the number of target entities encapsulated in a microcompartment, the method comprising the steps of:
- Providing a first detection molecule comprising (i) a target binding site specific for the target entity and (ii) a binding site capable of being bound by a second detection molecule;
- Providing a second detection molecule comprising (x) a target binding site specific for the first detection molecule, and (y) an enzymatic component capable of catalyzing a chemical signaling reaction;
- Bringing into contact the first- and the second detection molecule and the target entity to allow for a specific binding of the first detection molecule to the target entity and a specific binding of the second detection molecule to the first detection molecule;
- Optionally, removing at least any unbound detection molecules;
- Encapsulating one or more (an unknown number of) target entities bound with first and/or second detection molecules into one or more micro-compartments together with substrate molecules, wherein the substrate molecule is a substrate of the enzymatic component, and which when brought into contact with the enzymatic component renders the enzymatic component to catalyse the chemical signalling reaction; and
- Quantifying the detectable signal for each micro compartment and thereby quantifying the number of target entities encapsulated in each microcompartment.

It is understood that the alternative of the first aspect constitutes an indirect signal induction based on the use of intermediate binding molecules.

It shall be understood that a specific binding may be preferred in some embodiments, the invention however is also realized if the target binding site is at least capable of binding to the target entity. Hence, such binding may be also unspecific.

As used herein, the term microcompartment as applied in context of the invention defines any structure which can contain or encapsulate the target entities of the invention and maintain a spatial association. The microcompartments of the invention therefore serve as closed reaction chambers containing a solvent in which free target entities are capable of spatially associate with any of the herein described detection molecules, along with the substrate molecules. Microcompartments suitable for use according to the invention are conveniently achieved by micro-compartmentalization, which is a process of physically confining the any entities. Physical confinement can be achieved through the use of various micro-compartments, including microdroplets, (micro) fluidic plugs, microparticles, microwells and microvesicles. In certain preferred embodiments of all aspects of the invention, fluidic plugs are preferred as microcompartment. Plugs are generally understood to refer to compartments with larger dimensions compared to microdroplets.

Hence, "plugs", "fluidic plugs" or "microfluidic plugs", in accordance with the present invention may vary in shape, and for example may be spherical or non-spherical. The shape of the plug may be dependent of the shape of the channel (e.g., a plug may be a deformed sphere traveling in a rectangular channel). The plugs maybe in the form of plugs comprising an aqueous plug-fluid containing one or more reagents and/or one or more products formed from a reaction of the reagents, wherein the aqueous plug-fluid is surrounded by a non-polar or hydrophobic fluid such as an oil. The plugs may also be in the form of plugs comprising mainly a non-polar or hydrophobic fluid which is surrounded by an aqueous fluid. The plugs may be encased by one or more layers of molecules that comprise both hydrophobic and hydrophilic groups or moieties. The term "plugs" also includes plugs comprising one or more smaller plugs, that is, plugs-within-plugs. The relative amounts of reagents and reaction products contained in the plugs at any given time depend on factors such as the extent of a reaction occurring within the plugs. Preferably, plugs contain a mixture of at least two plug fluids. Preferred plugs of the invention are formed in three phase systems, such as a system consisting of aqueous plugs (composed of an aqueous phase) in fluorinated oil as a carrier phase, further separated by a mineral oil phase (droplets) acting as a second level spacer.

Forming of plugs of the invention may be formed in some embodiments in a substrate when a stream of at least one plug-fluid is introduced into the flow of a carrier-fluid in which it is substantially immiscible. The flow of the fluids in the device is induced by a driving force or stimulus that arises, directly or indirectly, from the presence or application of, for example, pressure, radiation, heat, vibration, sound waves, an electric field, or a magnetic field. Plugs in accordance with the present invention may vary in size but when formed, their cross-section should be substantially similar to the cross-section of the channels in which they are formed. When plugs merge or get trapped inside plug traps, the cross-section of the plugs may change. For example, when a plug enters a wider channel, its cross-section typically increases.

Generally, plugs may be formed in any microfluidic system and vary greatly in size and shape. For the present invention general dimensioning of plugs shall not be relevant and longitudinal and/or lateral diameter of a plug in accordance with the invention can range from mm dimensions to nm.

A "plug-fluid" refers to a fluid wherein or using which a reaction or precipitation can occur. Typically, the plug-fluid contains a solvent and most preferably is aqueous containing one or more substances such as any reagents, target entities, substrate molecules etc, in accordance with the invention. Although in some embodiments at least one plug-fluid may not contain one or more of the reagents of the invention. The reagent may be soluble or insoluble in the solvent. The plug-fluid may contain a surfactant. For example, a plug of the invention may be separated by one or more immiscible phases, such as mineral oil phases and/or fluorinated oils.

As used herein, the term microwell (used herein interchangeably with the term well) refers to a chamber having a volume of less than 1 ml. Microwells having dimensions in the nm range can be fabricated by electron beam lithography (see e.g. Odom et al. (2002) J. AM. CHEM. SOC. 124: 121 12-12113). Microwells are typically disposed on a solid substrate or microtiter plate (also referred to as a microplate, microwell or multiwall plate), this typically taking the form of a flat plate with multiple "wells" used as small test tubes. Microplates typically have 6, 24, 96, 384 or 1536 sample microwells arranged in a 2:3 rectangular matrix.

The term "microfluidic droplet" refers to an aqueous microcompartment of a certain size that encapsulates an aqueous liquid. The size of the microfluidic droplet is usually expressed as the diameter of the droplet when in a spherical shape. The diameter is generally between 20 and 400 µ 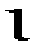 η, and preferably between 30 and 350 µ 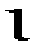 η, between 40 and 300 µ 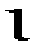 η, between 40 and 250 µ 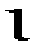 η, between 40 and 200 µ 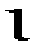 η or between 40 and 100 µ 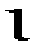 η (wherein each narrower range is preferred to the foregoing broader ranges and "between" includes the values mentioned). In a preferred embodiment, the diameter of the microfluidic droplet is between 2 and 20 times the diameter of the largest particle (e.g. the first particle or a further particle) in the droplet, preferably between 3 and 18 times, between 4 and 16 times, or between 5 and 14 times or between 6 and 12 times (wherein each narrower range is preferred to the foregoing broader ranges and "between" includes the values mentioned). Preferably, the diameter of the droplet is defined by both the above absolute and relative parameters. For example, the diameter is (i) between 20 and 400 µ 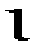 η, between 30 and 350 µ 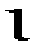 η, between 40 and 300 µ 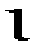 η, between 40 and 300 µ 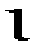 η, between 40 and 250 µ 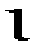 η, between 40 and 200 µ 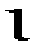 η or between 40 and 100 µ 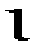 η and between 2 and 20 times the diameter of the largest particle (e.g. the first particle or a further particle) in the droplet, (ii) between 20 and 400 µ 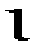 η, between 30 and 350 µ 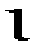 η, between 40 and 300 µ 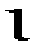 η, between 40 and 300 µ 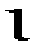 η, between 40 and 250 µ 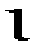 η, between 40 and 200 µ 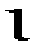 η or between 40 and 100 µ 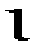 η and between 4 and 16 times the diameter of the largest particle (e.g. the first particle or a further particle) in the droplet, or (iii) between 20 and 400 µ 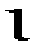 η, between 30 and 350 µ 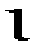 η, between 40 and 300 µ 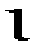 η, between 40 and 300 µ 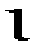 η, between 40 and 250 µ 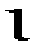 η, between 40 and 200 µ 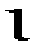 η or between 40 and 100 µ 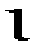 η and between 6 and 12 times the diameter of the largest particle (e.g. the first particle or a further particle) in the droplet.

Alternatively, the size of the microfluidic droplet can also be defined by volume. For example, it is usually less than 1 microlitre (µl). Preferably, it is less than 500 nanolitres (nl), less than 250, less than 150, less than 100 or less than 50 nl. In a preferred embodiment, it is between 0.05 and 150 nl, preferably between 0.05 and 125 nl, between 0.05 and 100 nl, between 0.05 and 80 nl, or between 0.05 and 4 nl (wherein each narrower range is preferred to the foregoing broader ranges and "between" includes the values mentioned).

A wide variety of compartmentalisation or microencapsulation procedures are available (Benita, S., Ed. (1996). Microencapsulation: methods and industrial applications. Drugs and pharmaceutical sciences. Edited by Swarbrick, J. New York: Marcel Dekker) and may be used to create the microfluidic droplet used in accordance with the present invention. Indeed, more than 200 microencapsulation or compartmentalisation methods have been identified in the literature (Finch, C. A. (1993) Encapsulation and controlled release. Spec. Publ.-R. Soc. Chem. 138, 35). These include membrane enveloped aqueous vesicles such as lipid vesicles (liposomes) (New, R. R. C, Ed. (1990). Liposomes: a practical approach. The practical approach series. Edited by Rickwood, D. & Hames, B. D. Oxford: Oxford University Press) and non-ionic surfactant vesicles (van Hal, D. A., Bouwstra, J. A. & Junginger, H. E. (1996). Nonionic surfactant vesicles containing estradiol for topical application. In Microencapsulation: methods and industrial applications (Benita, S., ed.), pp. 329-347. Marcel Dekker, NewYork.). Preferably, the microcompartments of the present invention are formed from emulsions; heterogeneous systems of two immiscible liquid phases with one of the phases dispersed in the other as droplets of microscopic size (Becher, P. (1957) Emulsions: theory and practice. Reinhold, New York; Sherman, P. (1968) Emulsion science. Academic Press, London; Lissant, K.J., ed Emulsions and emulsion technology. Surfactant Science New York: Marcel Dekker, 1974; Lissant, K.J., ed. Emulsions and emulsion technology. Surfactant Science New York: Marcel Dekker, 1984). Emulsions may be produced from any suitable combination of immiscible liquids. Preferably the emulsion of the present invention has water (containing a particle and other components) as the phase present in the form of droplets and a hydrophobic, immiscible liquid (preferably an oil) as the surrounding matrix in which these droplets are suspended. Such emulsions are termed 'water- in-oil'. This has the advantage that the aqueous phase is compartmentalised in discrete droplets. The external phase, preferably being a hydrophobic oil, generally is inert. The emulsion may be stabilized by addition of one or more surface- active agents (surfactants). These surfactants act at the water/oil interface to prevent (or at least delay) separation of the phases. Many oils and many emulsifiers can be used for the generation of water-in-oil emulsions; a recent compilation listed over 16,000 surfactants, many of which are used as emulsifying agents (Ash, M. and Ash, I. (1993) Handbook of industrial surfactants. Gower, Aldershot). Suitable oils are listed below.

The term "detection molecule" as used in context of the present invention shall preferably pertain to any molecular structure that is able to specifically bind to a target entity or to another detection molecule. As such detection molecules of the invention are preferably selected from antigen binding constructs such as antigen binding proteins or nucleic acids. Preferred are proteins or nucleic acid constructs that can specifically bind a target entity, and which allow either binding of a second detection molecule or allow for a coupling to an enzymatic component as described herein elsewhere. Preferably, the entity binding molecules that are used as detection molecules of the invention are selected from the group consisting of an antibody, an antibody derivative, an antibody mimetic, a bacteriophage, an mRNA-polypeptide complex, an mRNA-ribosome-polypeptide complex, a cell, a virus, a peptide, a protein, a nucleic acid, an aptamer and a small molecule.

The term "antibody" refers to both monoclonal and polyclonal antibodies, i.e. any immunoglobulin protein or portion thereof which is capable of recognizing an antigen or hapten, or any antigen binding derivative of fragments thereof. Exemplary antibodies are IgA, IgD, IgE, IgG, IgM, IgY or IgW. The term "antibody derivative" as used herein refers to a molecule comprising at least one antibody variable domain, but not having the overall structure of an antibody such as IgA, IgD, IgE, IgG, IgM, IgY or IgW, although still being capable of binding a target molecule. Said derivatives may be, but are not limited to functional (i.e. target binding, particularly specifically target binding) antibody fragments such as Fab, Fab2, scFv, Fv, or parts thereof, or other derivatives or combinations of the immunoglobulins such as nanobodies, diabodies, minibodies, camelid single domain antibodies, single domains or Fab fragments, domains of the heavy and light chains of the variable region (such as Fd, VL, including Vlambda and Vkappa, VH, VHH) as well as mini-domains consisting of two beta-strands of an immunoglobulin domain connected by at least two structural loops. Preferably, the antibody derivative is monovalent. More preferably, the derivative is a single chain antibody, most preferably having the structure VL-peptide linker- VH or VH-peptide linker- VL.

The term "antibody mimetic" as used herein refers to organic compounds that, like antibodies, can specifically bind antigens, but that are not structurally related to antibodies. They are usually artificial peptides or proteins with a molar mass of about 3 to 20 kDa. Non- limiting examples of antibody mimetics are affibodies, affilins, affimers, affitins, anticalins, avimers, DARPins, fynomers, Kunitz domain peptides, monobodies, Z domain of Protein A, Gamma B crystalline, ubiquitin, cystatin, Sac7D from Sulfolobus acidocaldarius, lipocalin, A domain of a membrane receptor, ankyrin repeat motive, SH3 domain of Fyn, Kunits domain of protease inhibitors, the 10th type III domain of fibronectin, synthetic heterobivalent or heteromultivalent ligands (Josan et al., Bioconjug Chem. 2011 22(7): 1270-1278; Xu et al., PNAS 2012 109 (52) 21295-21300; Shallal et al., Bioconjug Chem. 2014 25(2) 393-405) or synthetic or non-synthetic peptide ligands, e.g. from a (random) peptide library. Synthetic peptide ligands have non-naturally occurring amino acid sequences that function to bind a particular target molecule. Peptide ligands within the context of the present invention are generally constrained (that is, having some element of structure as, for example, the presence of amino acids which initiate a β turn or β pleated sheet, or for example, cyclized by the presence of disulfide bonded Cys residues) or unconstrained (linear) amino acid sequences of less than about 50 amino acid residues, and preferably less than about 40 amino acid residues. Of the peptide ligands less than about 40 or 50 amino acid residues, preferred are the peptide ligands of between about 10 and about 30 amino acid residues.

The term "bacteriophage" refers to a virus that infects and replicates in bacteria. In a preferred embodiment, the bacteriophage displays a peptide or protein heterologous (i.e. foreign) to the bacteriophage, which according to the present invention is the potential particle binding partner, therefore is the target entity or the first detection molecule. This embodiment is based on phage display, which is a technique for studying interactions with peptides or proteins displayed by the bacteriophage. In this technique, a gene encoding a protein of interest is inserted into a phage coat protein gene, causing the phage to display the protein on its outside while containing the gene for the protein on its inside, resulting in a connection between genotype and phenotype. These displaying phages can then be screened against the particle(s), in order to detect interaction between the displayed peptide or protein and the particle(s). In this way, large libraries of peptides or protein can be screened and amplified in a process called in vitro selection. Preferred bacteriophages are Ml₃ phage, fd filamentous phage, T₄ phage, T₇ phage and lambda phage.

The term "mRNA-polypeptide complex" refers to a complex for mRNA display. mRNA display is a display technique used for in vitro protein and/or peptide evolution to create molecules that can bind to a desired target. The process results in translated peptides or proteins that are associated with their mRNA progenitor via a puromycin linkage. The complex then binds to an immobilized target in a selection step. The mRNA -protein fusions that bind well are then reverse transcribed to cDNA and their sequence amplified via a polymerase chain reaction. The result is a nucleotide sequence that encodes a peptide with high affinity for the molecule of interest. See also Lipovsek and Pliickthun, Journal of Immunological Methods 290 (2004) 51 - 67.

The term "mRNA-ribosome -polypeptide complex" refers to a complex for ribosome display. ep. Ribosome display is a technique used to perform in vitro protein evolution to create proteins that can bind to a desired ligand, such as a target entity. The process results in translated proteins that are associated with their mRNA progenitor which is used, as a complex, to bind to an immobilized ligand in a selection. The mRNA-protein hybrids that bind well are then reversed transcribed to cDNA and their sequence amplified via PCR. The end result is a nucleotide sequence that can be used to create tightly binding proteins. See also Lipovsek and Pliickthun, Journal of Immunological Methods 290 (2004) 51 - 67.

The term virus refers to a small infectious agent replicating only inside living cells of a host organism. Preferably, it is a pathogenic virus, more preferably a virus pathogenic in mammals, in particular humans, e.g. from the class of herpesviridae, adenoviridae, papillomaviridae, polyomaviridae, poxviridae, anelloviridae, parvoviridae, reoviridae, coronaviridae, astroviridae, caliciviridae, flaviviridae, picornaviridae, togaviridae, rhabdoviridae, filoviridae, paramyxoviridae, arenaviridae, bunyaviridae, orthomyxoviridae, retroviridae or hapadnaviridae. Preferably, the potential particle binding partner is not a virus if the particle is a virus.

A protein as a potential detection molecule may be a natural or a recombinant protein. Preferably, other than proteins defined above such as antibodies, it is a protein displayed or present on the surface of a cell, a receptor or a receptor ligand.

A nucleic acid as a potential detection molecule may be a natural or a recombinant nucleic acid. Preferred nucleic acids are DNA, RNA and nucleic acid analogues thereof. Nucleic analogues are compounds which are structurally similar to naturally occurring RNA and DNA Nucleic acids are chains of nucleotides, which are composed of three parts: a phosphate backbone, a pucker-shaped pentose sugar, either ribose or deoxyribose, and one of four nucleobases. An analogue may have any of these altered. Typically, the analogue nucleobases confer, possibly among other things, different base pairing and base stacking properties. Examples include universal bases, which can pair with all four canon bases. Nucleic analogues, also termed artificial nucleic acids, include peptide nucleic acid (PNA), Morpholino, locked nucleic acis (LNA), glycol nucleic acid (GNA) and threose nucleic acid (TNA). Each of these is distinguished from naturally occurring DNA or RNA by changes to the backbone of the molecule. A preferred nucleic acid analogue is L-ribonucleic acid aptamer (L-RNA aptamer, trade name Spiegelmer), which is an RNA-like molecule built from L-ribose units. It is a mirror-image of natural oligonucleotides and a form of aptamer. Due to its L-nucleotides, it is highly resistant to degradation by nucleases.

The term "aptamer" refers to an oligonucleotide or peptide molecule that binds to a specific target molecule. Aptamers are usually created by selecting them from a large random sequence pool, but natural aptamers also exist in riboswitches.

The term "target binding site" shall be understood in its broadest sense and pertain to any surface structure or chemical moiety (or group) of an entity that allows for a specific (binding) interaction with another entity. Any specific binding, but also unspecific binding functionalities can be harnessed in accordance with the present invention to allow a "labelling" of the target entity with the detection molecule in accordance with the invention. As such a target binding site in context of the invention may be encoded by a nucleic acid sequence, or any epitope structure that can be bound by immunoglobulins. Typical binding interactions may be covalent or non-covalent, may include hydrogen bonds, van-der-Waals forces or hydrophobic interactions. A particular preferred antigen binding site is a functionality that allows a specific binding to cell membranes.

Any interaction between two entities that results in the formation of a binding interaction shall be considered for this invention. A "binding interaction" (sometimes shortened to "binding") refers to the interaction of a molecule of interest (e.g., a first entity) with another molecule (e.g., an second binding entity or). Such interaction partners may be understood as ligands and antiligands. Examples of ligand/antiligand binding interactions to which the present invention relates are (1) simple, non-covalent binding, such as dipole-dipole interactions, hydrogen bonding, or van der Waals interactions, and (2) temporary covalent bond formation, such as often occurs when an enzyme is reacting with its substrate. More specific examples of binding interactions of interest include, but are not limited to, ligand/receptor, antigen/antibody, enzyme/substrate, DNA/DNA, DNA/RNA, RNA/RNA, nucleic acid mismatches, complementary nucleic acids and nucleic acid/proteins. Binding interactions can occur as primary, secondary, or higher order binding interactions. A primary binding interaction is defined as a first molecule binding (specifically or non-specifically) to a second molecule to form a first molecular interaction complex; a secondary binding interaction is defined as a second molecule binding (specifically or non-specifically) to the first molecular interaction complex; and so on for higher order binding events. The product of ligand/antiligand binding is a ligand/antiligand complex.

Detection molecules, or first and/or second detection molecules, which are proteins are preferably antibodies or any antigen binding fragment or derivative of an antibody.

It is preferred that the methods of the first aspect are for the quantification of the number of encapsulated target entities of distinct species of target entities out of a plurality of species of target entities, wherein the method comprises the steps of
- Providing multiple species of detection molecules, each species of detection molecules comprising (i) a target binding site specific for a distinct species of target entity out of a plurality of species of target entities and (ii) an enzymatic component that is capable of catalysing a chemical signalling reaction that is different from a chemical signalling reaction catalysed by any other enzymatic component comprised in a detection molecule of another species of detection molecules;
- Bringing into contact the multiple species of detection molecules and the plurality of species of target entities to allow for a specific binding of the detection molecule to the target entities;
- Optionally, removing at least any unbound detection molecules;
- Encapsulating one or more (an unknown number of) target entities of each species of target entities bound with detection molecules into one or more micro-compartments together with multiple species of substrate molecules, wherein each species of substrate molecule is a substrate for the enzymatic component of not more than one species of detection molecule, and which when brought into contact with the corresponding enzymatic component, the enzymatic component catalyses the chemical signalling reaction that generates a distinct detectable signal for each of the chemical signalling reactions;
- detecting each detectable signal for each micro compartment and thereby quantifying the number of target entities of each species of target entities encapsulated in each microcompartment.

In addition, the aforementioned alternative aspect using a first and a second detection molecule can be applied for the embodiment of quantification of the number of encapsulated target entities of distinct species of target entities out of a plurality of species of target entities.

In preferred embodiments of the invention an enzymatic component or enzyme used is any protein (or also ribozyme) that allows for a detectable and quantifyable catalyzation of a chemical reaction, preferably which may be transformed into a any detectable signal. Typical and preferred examples of such enzymes are a peroxidase, such as horseradish peroxidase, cytochrome C peroxidase, glutathione peroxidase, microperoxidase, myeloperoxidase, lactoperoxidase, or soybean peroxidase, or is an alkaline phosphatase, or any enzymatically active fragment or derivative of these proteins. A further class of enzymes usable in context of the invention is a luciferase or a fragment thereof, such as Renilla luciferase, Gaussia luciferase, firefly luciferase, or Nanoluc, and wherein the substrate molecule is a luciferase substrate.

The substrate used in context of the invention naturally depends on the enzyme used. Typical substrates used are selected from 3-amino-9-ethylcarbazole, 3,3',5,5'-Tetramethylbenzidine, 4-chloro-11-naphthol, Fast Blue BB, NBT/BCIP, Fast Red, and AP-Fast Red, Amplex red.

A "target entity" of the invention, of which its encapsulation is quantified or controlled, refers to any object small enough to fit into the microfluidic compartment such as a droplet or well, and which can be bound by a detection molecule. Preferably, a target entity is selected from the group consisting of a cell, a bead, a virus, a macromolecule, such as a protein, nucleic acid, lipid or carbohydrate, and a nanoparticle. The cell can be any prokaryotic or eukaryotic cell. Preferably, it is a eukaryotic cell, e.g. a yeast cell, plant cell or animal cell. Animal cells include insect, nematode, fish and mammalian cells. More preferably it is mammalian cell, e.g. a mouse, rat, monkey or human cell. For example, it can be a random cell of a heterogeneous cell population (e.g. from a tissue) or it can be a specifically selected cell, selected, e.g., by FACS. Also, it can be a cell from cell line or a homogeneous culture, for example of a primary cell, wherein "primary" means derived directly from a tissue or organism and not manipulated to have altered properties, e.g. to divide indefinitely. Other examples for cells are developing cells, stem cells or cancer cells. Preferred examples of cells are patient samples (e.g. patient cancer cells), antibody-producing cells (e.g. B cells or hybridoma cells) and reporter cells for example signaling, preferably by fluorescence, the effect of the antibody produced by the antibody-producing cell on the reporter cell (e.g. the activation or inhibition of a receptor, preferably a G-protein coupled receptor). The "bead" (also termed "microbead") is a uniform polymer particle with a diameter of up to 1 micrometre, preferably of 0.5 to 500 µ 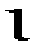 η, and with a surface to which biological entities such as cells, proteins including antibodies and/or nucleic acids as well as detectably labels as described below can bind or be coupled. The beads referred to herein are usually polyethylene or polystyrene beads or beads made of gel matrices, for example coated with protein/peptide/antigen, e.g. cellular antigen (e.g. a cell surface antigen).

For any particles in particular cells, it is preferably that the detection molecule may specifically bind to any specific surface structure, which can be e.g. surface markers/plasma membrane markers, such as specific markers for all particles/cells in the microcompartment or only for a subpopulation or a certain species if mixtures of species are present like in a biopsy or for samples from mouse xenograft models with implanted human tumors. For human cells the most sufficient detection molecules are antibodies e.g. β2-microglobulin antibody and anti-C298 antibody and for mouse cells anti-mouse H-2 antibody and Anti-mouse CD45 antibody each combined with a second detection molecule, such as for example a m-IgG BP-HRP antibody. Typical further antigens used as anchor detection molecules of the invention are alternative cluster of differentiation proteins (CDs), Anti-Sodium Potassium ATPase antibody, anti-CD98, Cadherin, Zonula, genetic probes, single strand RNA, peptide sequence or a chemical binding, e.g. carboxy group/aminogroup, azido/alkylene group and amine group, biotine-steptavidine or a heterobifunctional linker like MU-NHS, a chemically or mechanically cleavable or photo-switchable linker.

Also larger target entities can be encapsulated in the micro compartments of the invention. For example, encapsulation of larger particles into fluidic plugs as described above may be preferred. In context of the present invention a larger particle as target entity may be any biological accumulation of multiple cells or cell derived structures (bone etc), tissue sections or cubes, cell spheroids, embryos, microscopic animals such as nematodes, and the like. The present invention generally is not restricted to certain dimensions, as long as a compartmentalisation is and encapsulation is achieved.

The term "protein" as used herein indicates a polypeptide with a particular secondary and tertiary structure that can interact with another analyte and in particular, with other biomolecules including other proteins, DNA, RNA, lipids, metabolites, hormones, chemokines, and small molecules. In can be a natural (i.e. unaltered by man) or a recombinant protein. Preferably, other than proteins defined above such as antibodies, it is a cell surface- displayed molecule, a receptor or a receptor ligand.

The term "nanoparticle" used herein refers to a particle having a diameter of from about 1 to 1000 nm, preferably 1 to 100 nm. Components of the nanoparticle may include metal such as gold, silver, copper, aluminum, nickel, palladium, platinum, alloys thereof, a semiconductor material such as CdSe, CdS, In As, InP, or core/shell structures thereof, or organic particles such as particles made from organic polymer, lipids, sugars, or other organic materials, e.g. polystyrene, latex, acrylate, or polypeptide. Such organic particles may optionally contain some inorganic material; however, the amount of inorganic material is less than 50%, less than 25%, less than 10%, less than 5%, or less than 1%.

An enzymatic reaction which is detectable or causes a detectable signal, is preferably a a colorimetric, chemiluminescent or fluorescent reaction.

In a preferred embodiment of the invention, in order to provide a robust quantification of the amount of encapsulated target entity, the substrate molecule is encapsulated within each microcompartment in excess to the concentration of the detection molecule in the same microcompartment. In excess means a concentration of the substrate molecule in the microcompartment which avoids that the chemical reaction will reach equilibrium, or consume all substrate molecules within one compartment, at a sub-maximum amount of target entity within the compartment.

In another preferred embodiment, each target entity when encapsulated is capable of eliciting one or more detectable assay signals, and wherein the method comprises in step (e) detecting the detectable assay signal, and normalizing the detectable assay signals of two more microcompartments with the detectable signals for each of the two or more microcompartments.

In **the second aspect,** the invention pertains to a plurality of microcompartments, of which at least 5% of microcompartments have an aqueous phase comprising a target entity bound with detection molecules, wherein the detection molecule comprises (i) a target binding site specific for the target entity and (ii) an enzymatic component that is capable of catalysing a chemical signalling reaction; and an unbound substrate molecule dissolved within the aqueous phase and which is a substrate of the enzymatic component of the detection molecule, and which when brought into contact with the enzymatic component renders the enzymatic component to catalyse the chemical signalling reaction that generates a detectable signal.

In **an alternative second aspect,** the invention pertains to a plurality of microcompartments, of which at least 5% of microcompartments have an aqueous phase comprising a target entity bound with first- and/or second detection molecules, wherein the first detection molecule comprises (i) a target binding site specific for the target entity and (ii) a binding site capable of being bound by a second detection molecule, and wherein the second detection molecule comprises (x) a target binding site specific for the detection molecule, and (y) an enzymatic component capable of catalysing a chemical signalling reaction; and an unbound substrate molecule dissolved within the aqueous phase and which is a substrate of the enzymatic component of the second detection molecule, and which when brought into contact with the enzymatic component renders the enzymatic component to catalyse the chemical signalling reaction that generates a detectable signal.

The term "population" or "plurality" means "more than one". In preferred embodiments, it means more than 10, more than 20, more than 30, more than 40, more than 50, more than 100, more than 250, more than 500 or more than 1000.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

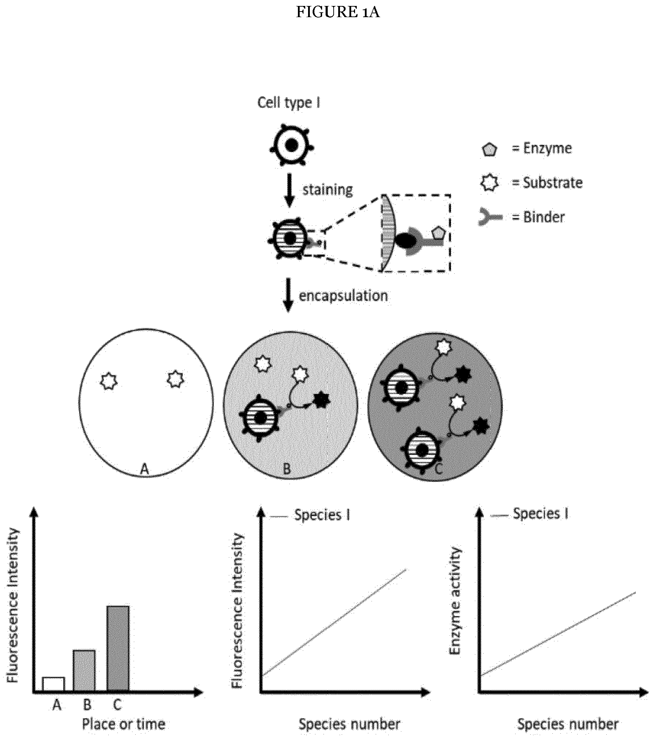

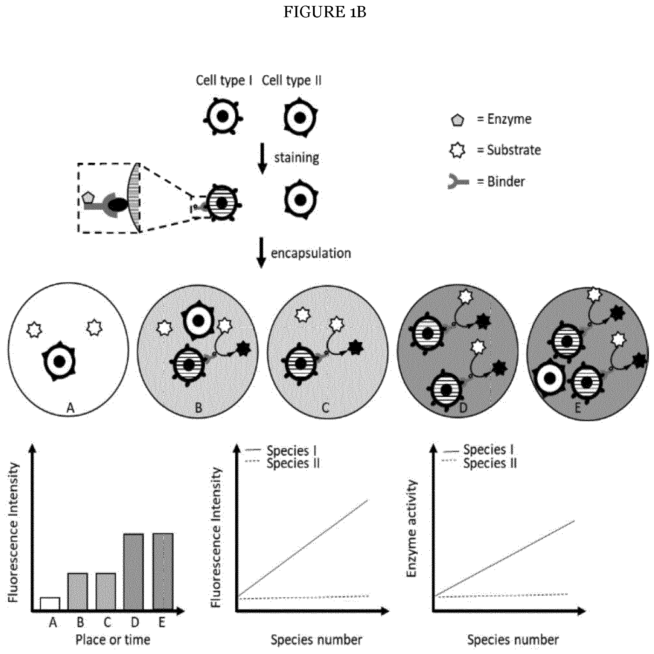

**Figure 1****:** shows the working principle of the procedure of the invention. **a)** embodiment of the invention with a single type of encapsulated entity of cell: cells to be encapsulated are stained with a binder directed to an expressed surface antigen (top schematic). The bottom schematic shows exemplary three separate possible compartment states after encapsulation. Version A shows a situation where no cell is encapsulated, version B shows a situation where only one cell is encapsulated, and version C shows the situation where two cells are encapsulated. Below the schematic compartments, the expected fluorescent signal is shown in the below prophetic graphs. **b)** embodiment of the invention similar to **a),** but with two different types of encapsulated entities (cells).

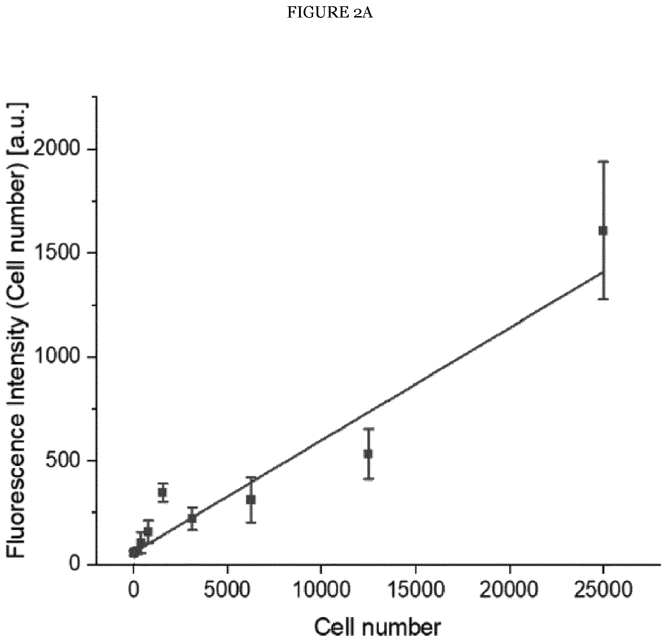

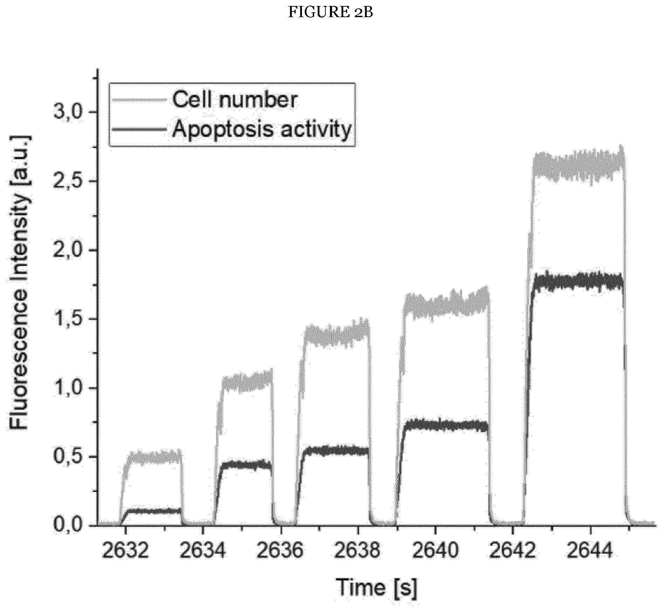

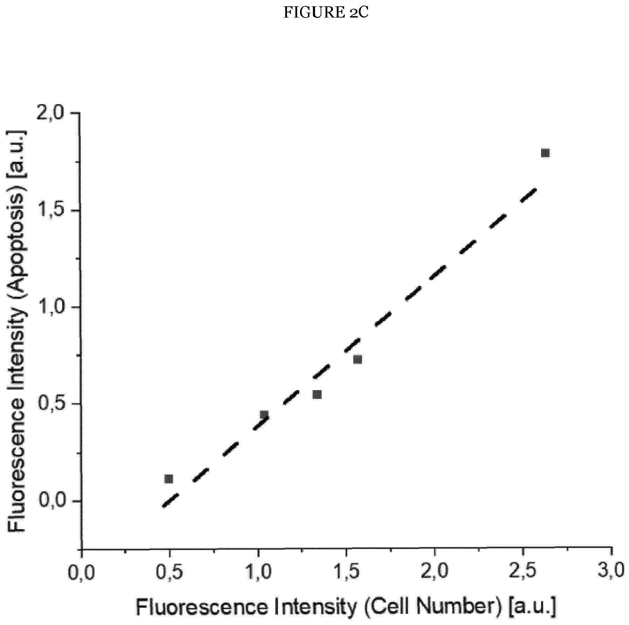

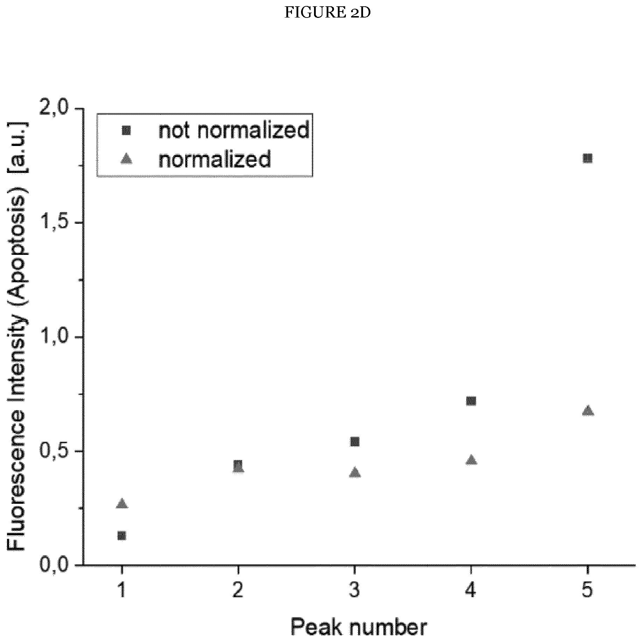

**Figure 2****:** shows the experimental testing of the method of the invention using an existing microfluidic personalized medicine platform described by previously (Eduati F, et al. (2018) Nature Communications 9(1):1-13.). **a)** the fluorescence signal in the microcompartment increases linear with the number of encapsulated cells. **b)** shows an example for a readout of the assay. Each compartment consists of cells which are co-encapsulated with drugs and two substrates one for caspase 3 indicating apoptosis (wavelength I) and substrate 2 (emission at wavelength II not equal to wavelength I), which reacts with the antibody-coupled enzymes on the cell membrane enabling to quantify the cell number in each compartment. **c)** The encapsulated drug kills the cells and the fluorescence signal of the cells undergoing apoptosis increases linear with the fluorescence intensity of substrate II for cell counting. Thus, apoptosis signal is linear with the number of cells. **d)** The apoptosis signal can be normalized to the number of cells in the microcompartments (= peaks in b)).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### The examples show:

### Example 1: Normalization of encapsulated cell numbers in microfluidic screens

The invention enables the normalization to the cell number for cell assays, e.g. caspase readout in plates and high-throughput droplet microfluidic experiments. Furthermore, it is describing the established procedure to dissect primary colorectal cancer biopsies (from colon, rectum and liver metastasis) using human tumor dissection kit (miltenyi) and the approach to combine caspase 3 with caspase 9 substrate for obtaining a Caspase readout indicating which of the tested drugs and combinations thereof show the strongest effect of killing the screened cancer cells. The general principle of the method of the invention is depicted in Figure 1.

In brief, cells are stained with a binder directed to an expressed surface antigen. The binder can be an antibody or antibody combination, which is functionalized with an enzyme. Unbound binder is removed by washing. In the next step, stained cells and the reporter substrate are co-encapsulated into aqueous microcompartments and incubated. Upon reaction of the substrate and enzyme the fluorescence intensity changes. For non-endpoint measurements, this change in fluorescence intensity is linear with the number of cells due to linearity between enzyme activity and species number (Figure 1A).

In addition, the cell quantification assay can also be applied to quantify the number of a specific cell type I within a mixture of different types of cells, when one subpopulation does not bind the enzyme-linked binder due to the fact, that the target-antigen is not expressed or due to the specificity of the binder to a certain species. Cells out of a mixture with a cell type II from another species will not contribute to the fluorescence signal unless the cells of cell type II are labelled with the same enzyme or another enzyme converting another substrate, allowing to count both cell types in a multiplexed fashion (Figure 1B).

In order to test the invention, the following assay-specific reagents were used:

Reagents amplex red assay
- Amplex red (Thermofisher, Art.no. A12222_5Mg)
- DMSO
- β2-microglobulin antibody (Biolegend, clone 2M2, Art.no. 316305)
- anti-C298 antibody (Biolegend, clone LNH-94, Art.no. 341704)
- m-IgG BP-HRP (Santa Cruz Biotechnology, Art.no. sc-516102)
- anti-mouse H-2 antibody (Biolegend, clone M1/42, Art.no. 125502)
- anti-mouse CD45 antibody (Biolegend, clone 30-F11, Art.no. 103101)
- HRP Goat anti-rat IgG antibody (Biolegend, clone Poly4054, Art.no. 103101)
- example 1:

Reagents caspase assay
- caspase-3 substrate (Z-Asp-Glu-Val-Asp)z-Rhodamine110 (BACCHEM, Art.no. 4048381.0025)
- caspase-9 substrate (AC-Leu-Glu-His-Asp-AFC trifluoroacetate salt, BACCHEM, Art.no. 4028699.0005)
- DMSO (Sigma Aldrich)
- 5X Reaction buffer (50mM PIPES, pH 7.4, 10mM EDTA, 0.5% CHAPS all from Sigma)
- DTT (1 M, Cleland's reagents, Roche, Art.no. 10197777001)
- Distillated water (dH2O)
- Cascade blue TM hydrazide tritium salt (10mM in DMSO) (Art.no. C3239, Invitrogen)
- Mineral oil (Sigma, Art.no. M8410-1L)
- 3MTM Fluorinated FC-40 (IOLITEC Ionic liquids technology GmbH)
- 1H,1H12H,2H-Perfluorooctanol (PFO, Abcr GmbH, Art.no. AB125017)

After preparation of the microfluidic setup, the cells for encapsulation the first detection antibody is added to the cells to allow binding to the cell surface (see **Table 1**) and incubate for 15 min at 4°C to allow for a sufficient surface staining. Then the stained cells are washed to remove unbound antibody. As a second step a HRP conjugated secondary antibody specific for the first antibody is added to the stained cells and incubated

**Table 1: Successful staining protocol of the cell membrane in example 1**

| **Species of cell origin** | **Add á 2 nl first antibody to 1 ml staining buffer** | **washing step** | **Add 1 µl secondary antibody to 1 ml staining buffer** | **washing step** |
|---|---|---|---|---|
| **Mouse** | anti-mouse H-2 antibody anti-mouse CD45 antibody | 14 ml staining buffer | Anti-rat-HRP | 14 ml staining buffer |
| **Human** | anti-human C298 antibody anti-human β2-microglobulin | 14 ml staining buffer | Anti- mouse- HRP | 14 ml staining buffer |
| **Time [min]** | 15 | 5 | 15 | 5 |

The stained cells are then encapsulated and signal detection is performed as described before (Eduati et al. 2018 Nature Communications volume 9, Article number: 2434, of which the "Methods" section is incorporated herein by reference in its entirety). Results of normalized signals are shown in Figure 2.

### Example 2: [prophetic] Functional antibody screens

In general, all assays carried out in either large aqueous compartments or compartments with multiple cells require a normalization for the number of cells. As another example the same kind of assay could be used for functional antibody screens involving more than one target cell: Single antibody expressing cells (e.g. plasma cells) can be encapsulated together with multiple target cells (e.g. cancer cells) into aqueous compartments for identifying antibodies that can trigger apoptosis in the target cells. In this type of assay one could stain the target cells with an enzyme-linked antibody to enable normalization of the functional antibody assay (e.g. induction of intracellular Ca-flux upon agonistic action of antibodies on GPCRs) to the number of cells per compartment. Only this way, the most potent therapeutic antibodies can be isolated (note that antibody discovery is a multi-billion dollar business in which even slight technical improvements have significant commercial impact).

### Example 3: [prophetic] Multiplex Screens

Assays involving different cell types (multiplex screens) are even more prone to biases based on cell number variation (and variation of the relative cell numbers). For example, assays in cancer immunology often include T-cells, target cells and antibody presenting cells. For being able to carry out such types of assays at high throughput in aqueous compartments, one has to be able to measure the relative cell numbers accurately, which can be achieved using the described assay. A different enzyme labelled antibody can be used for each cell type (particularly with a different enzyme for each cell type) so that the relative cell numbers can be easily detected by determining the substrate turnover for the different enzymes (e.g. using substrate-i for enzyme-1, conjugated to antibodies used for staining cell type 1, substrate-2 for enzyme-2, conjugated to antibodies used for staining cell type 2, etc.)

## Claims

1. **A method for the quantification of the number of target entities encapsulated in a micro compartment,** the method comprising the steps of:
**(a)** Providing a detection molecule comprising (i) a target binding site specific for the target entity, or at least capable of binding to the target entity, and (ii) an enzymatic component capable of catalysing a chemical signalling reaction;
**(b)** Bringing into contact the detection molecule and the target entity to allow for a specific binding of the detection molecule to the target entity;
**(c)** Optionally, removing at least any unbound detection molecules;
**(d)** Encapsulating one or more (an unknown number of) target entities bound with detection molecules into one or more micro-compartments together with substrate molecules, wherein the substrate molecule is a substrate of the enzymatic component, and which when brought into contact with the enzymatic component renders the enzymatic component to catalyse the chemical signalling reaction; and
**(e)** Quantifying the detectable signal for each micro compartment and thereby quantifying the number of target entities encapsulated in each micro compartment.

2. The method of claim 1, which is for the quantification of the number of encapsulated target entities of distinct species of target entities out of a plurality of species of target entities, wherein the method comprises the steps of
**(a)** Providing multiple species of detection molecules, each species of detection molecules comprising (i) a target binding site specific for a distinct species of target entity out of a plurality of species of target entities, or at least capable of binding to a target entity our of a plurality of species of target entites and (ii) an enzymatic component that is capable of catalysing a chemical signalling reaction that is different from a chemical signalling reaction catalysed by any other enzymatic component comprised in a detection molecule of another species of detection molecules;
**(b)** Bringing into contact the multiple species of detection molecules and the plurality of species of target entities to allow for a specific binding of the detection molecule to the target entities;
**(c)** Optionally, removing at least any unbound detection molecules;
**(d)** Encapsulating one or more (an unknown number of) target entities of each species of target entities bound with detection molecules into one or more micro-compartments together with multiple species of substrate molecules, wherein each species of substrate molecule is a substrate for the enzymatic component of not more than one species of detection molecule, and which when brought into contact with the corresponding enzymatic component, the enzymatic component catalyses the chemical signalling reaction that generates a distinct detectable signal for each of the chemical signalling reactions;
**(e)** detecting each detectable signal for each micro compartment and thereby quantifying the number of target entities of each species of target entities encapsulated in each micro compartment.

3. **A method for the quantification of the number of target entities encapsulated in a micro compartment,** the method comprising the steps of:
**(a)** Providing a first detection molecule comprising (i) a target binding site specific for the target entity and (ii) a binding site capable of being bound by a second detection molecule;
**(b)** Providing a second detection molecule comprising (x) a target binding site specific for the first detection molecule, and (y) an enzymatic component capable of catalyzing a chemical signaling reaction;
**(c)** Bringing into contact the first- and the second detection molecule and the target entity to allow for a specific binding of the first detection molecule to the target entity and a specific binding of the second detection molecule to the first detection molecule;
**(d)** Optionally, removing at least any unbound detection molecules;
**(e)** Encapsulating one or more (an unknown number of) target entities bound with first and/or second detection molecules into one or more micro-compartments together with substrate molecules, wherein the substrate molecule is a substrate of the enzymatic component, and which when brought into contact with the enzymatic component renders the enzymatic component to catalyse the chemical signalling reaction; and
**(f)** Quantifying the detectable signal for each micro compartment and thereby quantifying the number of target entities encapsulated in each micro compartment.

4. The method of any one of claims 1 to 5, wherein the target entity is a particle, such as a micro or nano particle, a bead, a vesicle, a biological cell, or a cell-accumulation, such as a tissue fragment, spheroid or organism, in particular an embryo or microscopic multicellular organism (worm, plant, fungus, etc.).

5. The method of any one of claims 1 to 4, wherein the enzymatic reaction is a colorimetric, chemiluminescent or fluorescent reaction.

6. The method of any one of claims 1 to 5, wherein the micro compartment is an aqueous micro compartment such as a plug, a (microfluidic) droplet or a well.

7. The method of any one of claims 1 to 6, wherein the substrate molecule is encapsulated within each micro compartment in excess to the concentration of the detection molecule in the same micro compartment.

8. The method of any one of claims 1 to 7, wherein each target entity when encapsulated is capable of eliciting one or more detectable assay signals, and wherein the method comprises in step (e) detecting the detectable assay signal, and normalizing the detectable assay signals of two more micro compartments with the detectable signals for each of the two or more micro compartments.

9. **A plurality of micro compartments,** of which at least 5% of micro compartments have an aqueous phase comprising a target entity bound with detection molecules, wherein the detection molecule comprises (i) a target binding site specific for the target entity and (ii) an enzymatic component that is capable of catalysing a chemical signalling reaction; and an unbound substrate molecule dissolved within the aqueous phase and which is a substrate of the enzymatic component of the detection molecule, and which when brought into contact with the enzymatic component renders the enzymatic component to catalyse the chemical signalling reaction that generates a detectable signal.

10. The plurality of micro compartments of claim 9, wherein the target entity is a particle, such as a micro or nano particle, a bead, a vesicle, a biological cell, or a cell-accumulation, such as a tissue fragment, spheroid or organism, in particular an embryo or microscopic multicellular organism (worm, plant, fungus, etc.).

11. The plurality of micro compartments of claim 9 or 10, wherein the enzymatic reaction is a colorimetric, chemiluminescent or fluorescent reaction.

12. The plurality of micro compartments of any one of claims 9 to 11, wherein the enzymatic component or enzyme is a peroxidase, or a fragment thereof, or a luciferase or a fragment.

13. The plurality of micro compartments of any one of claims 9 to 12, wherein the substrate molecule is encapsulated within each micro compartment in excess to the concentration of the detection molecule in the same micro compartment.

14. The plurality of micro compartments of any one of claims 14 to 22, wherein the micro compartment is an aqueous micro compartment such as a plug, a (microfluidic) droplet or a well

15. The plurality of micro compartments of claim 14, wherein the micro compartment is a plug formed in a three-phase system preferably composed of an aqueous phase, a fluorinated oil phase as a carrier phase, and a mineral oil phase as a spacer between individual plugs of the plurality of micro compartment.
